# FASCICULE DE BREVET EUROPEEN

(11) **EP 4 229 030 B1**
(45) Date de publication et mention de la délivrance du brevet: **05.11.2025**
(21) Numéro de dépôt: 21805568.9
(22) Date de dépôt: 14.10.2021
(51) Int. Cl.: C07C 229/12, C07C 227/18, C11D 1/90

(54) **MÉLASSE FERMENTÉE ET ESTÉRIFIÉE**
FERMENTIERTE UND VERESTERTE MELASSE
FERMENTED AND ESTERIFIED MOLASSES

(30) Priorité: 15.10.2020 FR 2010571
(43) Date de publication de la demande: 23.08.2023
(73) Titulaire: Lesaffre et Compagnie, 75001 Paris (FR)
(72) Inventeur: LEBRUN, Xavier, 59520 MARQUETTE-LEZ-LILLE (FR)
(74) Mandataire: Plasseraud IP
(86) Numéro de dépôt international: PCT/FR2021/051792
(87) Numéro de publication internationale: WO 2022/079394

(56) Documents cités:
- WO-A1-2013/188508
- FR-A1- 3 082 522
- FR-A1- 3 088 930

## Description

### Domaine technique

La présente invention se rapporte au domaine de l'industrie sucrière et concerne particulièrement une nouvelle mélasse fermentée, son procédé de préparation et son utilisation en tant qu'agent d'amélioration des propriétés tensioactives et/ou émulsifiantes d'une composition.

### Technique antérieure

La glycine bétaïne (C₅H₁₁NO₂) est une molécule à partir de laquelle peut être obtenue une multitude de dérivés qui trouvent des applications diverses et variées, notamment dans le domaine des tensioactifs.

En effet, la glycine bétaïne, également appelée triméthylglycine, est une substance naturelle peu onéreuse qui constitue une matière première de choix pour la préparation d'agents tensioactifs. A titre d'exemple, les alkyl de bétaïne ou les amidoalkyl de bétaïne sont les plus largement utilisés.

Le document WO 2015/078890 décrit des compositions d'esters gras de glycine bétaïne, des esters de glycine bétaïnes et d'alkyl polyglucosides qui renferment notamment des structures de type alkyl polyglucosides porteurs d'un groupement cationique introduit grâce à un greffage de glycine bétaïne.

Le document FR 2 869 913 propose par exemple des voies d'accès à base d'ester ou d'amide de la glycine bétaïne, obtenus sous forme de bruts réactionnels ou par lavage des bruts réactionnels par des solvants organiques.

Le document WO 2013/188508 décrit des compositions contenant des esters et/ou des amides de glycine bétaïne cationiques. Les alkylène bétaïnate méthane sulfonates et les bétainyl amino alkylène méthane sulfonates faisant partie de ces esters et de ces amides. Les esters et les amides de glycine bétaïne servent dans lesdites compositions de tensioactifs cationiques à activité antimicrobienne et sont présentés comme étant efficaces en tant que mélanges bruts ou semi-purifiés ou purifiés.

Le document FR 3 082 52 décrit une composition tensioactive comprenant un sel d'amide de glycine bétaïne, un sel d'alkylammonium, un sel d'ester de glycine bétaïne et de la glycine bétaïne.

Le document FR3 088 930 décrit l'utilisation de mélanges comprenant des esters de glycine bétaïne pour améliorer les propriétés tensioactives des compositions, lesdits esters de glycine bétaïne étant obtenus par réaction de la glycine glycine bétaïne avec un alcool gras.

Bien qu'il existe déjà un certain nombre de dérivés de glycine bétaïne, il reste nécessaire de proposer d'autres alternatives pouvant améliorer les propriétés tensioactives et/ou émulsifiantes, et notamment des solutions vertes, dites respectueuses de l'environnement, par opposition aux solutions d'origines pétrochimiques bien trop souvent employées.

D'une manière plus générale, la glycine bétaïne est un sous-produit de l'industrie sucrière et se retrouve dans la mélasse de betterave à sucre dont elle représente environ 5% à 7% en poids de matière sèche par rapport au poids total de matière sèche de ladite mélasse de betterave.

Comme précédemment mentionnée, la glycine bétaïne est une matière première de choix pour la préparation d'agents tensioactifs.

Jusqu'à présent, elle est extraite par les industriels directement à partir de la mélasse de betterave au travers par exemple de procédés chromatographiques. La glycine bétaïne ainsi extraite est concentrée puis utilisée notamment pour l'obtention de dérivés, comme décrit précédemment.

Le document WO 2004/002938 décrit par exemple un procédé de fractionnement chromatographique suivi d'une nanofiltration, ledit procédé permettant ainsi de récupérer au moins une fraction enrichie en glycine bétaïne à partir d'une solution de départ comprenant de la bétaïne, par exemple une solution de mélasse.

La mélasse est une substance connue de l'homme du métier. Il s'agit d'un coproduit issu de la fabrication du sucre à partir de la betterave et de la canne en sucrerie, ou des sucres roux en raffinerie. Le processus de fabrication du sucre, que celui-ci soit fait à partir de canne ou de betterave, aboutit après l'étape de cristallisation à l'obtention du sucre d'une part et de la mélasse d'autre part.

Bien qu'utilisée pour extraire la glycine bétaïne, la mélasse de betterave est plus généralement employée pour l'alimentation animale, en mélange avec de la paille ou d'autres aliments cellulosiques, mais également comme liant dans les rations complètes animales, ou encore pour favoriser chez l'animal l'ingestion d'aliments peu appétibles.

En alternative de l'alimentation animale, la mélasse est également utilisée par les industriels pour la production de produits dits « nobles » via des processus de fermentation. En effet, par l'intermédiaire des mécanismes de fermentation dont disposent certains micro-organismes, la mélasse peut servir de substrat et permet notamment l'obtention de levure boulangère, d'alcool éthylique, d'acides citrique et glutamique, de lysine ou encore d'antibiotiques.

En contrepartie, l'utilisation de la mélasse via les processus de fermentation génère de grandes quantités de résidus liquides de fermentation. Ces résidus liquides de fermentation correspondent à la mélasse dite fermentée.

Ayant été appauvrie en constituants par les micro-organismes, la mélasse fermentée est globalement considérée comme un résidu de fermentation présentant un faible intérêt, et se retrouve principalement valorisée dans le domaine de l'agriculture en tant qu'engrais d'épandage.

Pour extraire la glycine bétaïne, la mélasse fermentée n'est pas considérée comme un produit de choix par les industriels en raison des grandes quantités d'eau nécessaires pour son extraction mais également en raison du coût des installations chromatographiques à mettre en œuvre pour réaliser l'extraction.

Cependant, au regard des quantités de mélasses fermentées produites chaque année, il existe également un besoin de proposer de nouvelles voies de valorisation de ce résidu de fermentation considéré jusqu'alors comme un coproduit ne présentant que peu d'attrait, et avantageusement de proposer également une alternative plus verte aux tensioactifs d'origines pétrochimiques.

Il est ainsi du mérite de la demanderesse d'avoir pu répondre à ce double objectif en proposant un nouveau procédé de préparation à partir de mélasse fermentée comme produit de départ.

### Résumé

La présente invention concerne ainsi un procédé de préparation d'une mélasse fermentée comprenant au moins un ester de glycine bétaïne, ledit procédé comprenant les étapes suivantes de :
1) fourniture d'une mélasse fermentée de betterave,
2) ajout dans ladite mélasse fermentée de betterave d'au moins un acide selon un ratio molaire acide/glycine bétaïne compris entre 1 et 2,2,
3) estérification de la mélasse fermentée acidifiée obtenue à l'étape précédente par mélange avec au moins un alcool.

L'invention concerne également une mélasse fermentée et estérifiée comprenant des esters de bétaïne ainsi que son utilisation pour améliorer les propriétés tensioactives et/ou émulsifiantes d'une composition.

### Description des modes de réalisation

Comme précédemment mentionné, la mélasse fermentée est considérée comme un résidu de fermentation principalement valorisée dans le domaine de l'agriculture et de l'élevage en tant qu'engrais d'épandage ou en alimentation animale.

La présente invention propose ainsi une nouvelle voie de valorisation de la mélasse fermentée par l'intermédiaire du procédé décrit ci-après.

Un premier objet de l'invention concerne donc un procédé de préparation d'une mélasse fermentée comprenant au moins un ester de glycine bétaïne, ledit procédé comprenant les étapes suivantes de :
1) fourniture d'une mélasse fermentée de betterave,
2) ajout dans ladite mélasse fermentée de betterave d'au moins un acide selon un ratio molaire acide/glycine bétaïne compris entre 1 et 2,2,
3) estérification de la mélasse fermentée acidifiée obtenue à l'étape précédente par mélange avec au moins un alcool.

D'une manière tout à fait surprenante, la Demanderesse a constaté que la mélasse fermentée de betterave pouvait être utilisée pour mettre en œuvre une réaction d'estérification afin d'obtenir au moins un ester de glycine bétaïne.

La mélasse fermentée de betterave comprend principalement de l'eau. Cette caractéristique constituait jusqu'alors une contrainte technique rédhibitoire à son utilisation directe comme milieu de réaction pour l'obtention d'ester de bétaïne.

En effet, ces quantités importantes d'eau dissuadaient l'homme du métier de mettre en œuvre une quelconque réaction d'estérification, et de surcroit pour estérifier les quantités de glycine bétaïne présentes dans la mélasse fermentée de betterave.

Ainsi, allant à l'encontre d'un préjugé technique et à l'inverse de ce qui était jusqu'à présent réalisé dans les domaines des tensioactifs, la Demanderesse a démontré que des esters de glycine bétaïne pouvaient être obtenus sans qu'il soit nécessaire d'extraire la glycine bétaïne, et directement à partir de la mélasse fermentée en l'utilisant comme milieu de réaction.

En effet, dans le domaine particulier des tensioactifs, les industriels ont principalement eu recours à l'extraction de la glycine bétaïne à partir de mélasse de betterave, puis à la mise en œuvre de différentes réactions sur la glycine bétaïne extraite de manière à obtenir les dérivés de bétaïne, notamment les esters de glycine bétaïne.

De plus, comme précédemment mentionné, la mélasse fermentée n'est pas un produit de choix pour l'extraction de la glycine bétaïne à grande échelle en raison des quantités d'eau importantes nécessaires pour son extraction.

En proposant un procédé de préparation qui permet d'estérifier directement la glycine bétaïne au sein de la mélasse fermentée, sans extraction préalable, de manière à obtenir des esters de glycine bétaïne, la Demanderesse va à l'encontre des pratiques classiquement mises en œuvre dans le domaine des tensioactifs.

La mélasse fermentée comprenant un ou plusieurs esters de glycine bétaïne obtenue en fin du procédé selon l'invention peut alors directement être utilisée pour améliorer des propriétés tensioactives et/ou émulsifiantes d'une composition.

D'une manière tout à fait avantageuse, le procédé de préparation selon l'invention permet ainsi d'exploiter et de recycler une partie des volumes importants de mélasse fermentée produits par les industriels en réalisant une estérification in situ de la glycine bétaïne encore présente dans ladite mélasse fermentée.

Le procédé de préparation selon l'invention permet ainsi de proposer une voie de valorisation de la mélasse fermentée et d'obtenir un nouveau coproduit trouvant une application particulièrement intéressante pour améliorer les propriétés émulsifiantes et/ou tensioactives d'une composition.

Le procédé de préparation selon l'invention comprend une première étape de fourniture de mélasse fermentée de betterave.

Comme précédemment mentionné, la mélasse fermentée est un coproduit de la mélasse obtenu après fermentation de cette dernière par des bactéries, des levures ou des champignons, ladite fermentation permettant d'obtenir des produits dits « nobles » tels que la levure boulangère, l'alcool éthylique ou encore l'acide citrique et glutamique.

Généralement, la mélasse fermentée peut être obtenue à partir d'une mélasse de betterave ou d'une mélasse de canne.

Selon l'invention, la mélasse fermentée est obtenue à partir de mélasse de betterave car la mélasse de canne ne comprend pas de glycine bétaïne. La mélasse fermentée selon l'invention est donc une mélasse fermentée de betterave.

De préférence, la mélasse fermentée de betterave est obtenue via la fermentation de la mélasse de betterave par des levures.

Selon un mode de réalisation particulier, la mélasse fermentée de betterave peut également être un mélange de mélasse fermentée de betterave et de mélasse fermentée de canne. Dans un tel mélange, la glycine bétaïne est alors apportée par la mélasse fermentée de betterave.

Selon ce mode de réalisation particulier, le mélange peut contenir jusqu'à 70 % en poids de mélasse fermentée de betterave, jusqu'à 80 % en poids de mélasse fermentée de betterave, jusqu'à 90 % en poids de mélasse fermentée de betterave, voire 95 % en poids de mélasse fermentée de betterave. Le reste étant constitué de mélasse fermentée de canne.

Comme précédemment mentionnée, la mélasse fermentée contient principalement de l'eau, à savoir plus de 90% en poids par rapport au poids total. Cette quantité importante d'eau a jusqu'à présent constituée un obstacle technique qui dissuadait les industriels de l'utiliser pour des réactions d'estérification.

Avantageusement, la mélasse fermentée peut être concentrée afin de réduire la quantité d'eau et d'obtenir des taux de matières sèches plus importants.

Selon un mode de réalisation particulier la mélasse fermentée peut être concentrée jusqu'à l'obtention d'un taux de matière sèche compris de 45 % à 80 %. De préférence, le taux de matière sèche de la mélasse fermentée est de 50 % à 75 %, et tout particulièrement de 55 % à 65 %, comme par exemple environ 60 %.

Selon un autre de mode de réalisation particulier, la mélasse fermentée de betterave fournie selon la première étape du procédé est une mélasse fermentée déminéralisée. La déminéralisation peut par exemple consister en une précipitation des sels de sulfate de potassium (K₂SO₄), sulfate de sodium (Na₂SO₄), sulfate de magnésium (MgSO₄) et sulfate de calcium (CaSO₄) par ajout d'acide sulfurique.

De manière avantageuse, la déminéralisation permet d'augmenter la proportion de la matière organique au sein de la mélasse fermentée et d'augmenter la proportion de glycine bétaïne par rapport à la matière sèche totale dans la mélasse fermentée.

Classiquement, puisqu'elle est destinée ou était utilisée en tant qu'engrais et dans l'alimentation animale, la mélasse fermentée de betterave peut également être définie par sa répartition en matières azotées et par son aminogramme.

Par conséquent, la mélasse fermentée selon l'invention peut ainsi présenter une répartition des matières azotées comme ci-après :
- azote des acides aminés totaux déterminés par la méthode de Kjeldahl : 25 % à 50 % de l'azote total,
- azote de bétaïne : 40 % à 50 % de l'azote total,
- azote ammoniacal : 2 % à 3 % de l'azote total.

Le procédé de préparation selon l'invention comprend ensuite une étape d'ajout dans la mélasse fermentée d'au moins un acide selon un ratio molaire acide/glycine bétaïne compris entre 1 à 2,2.

L'homme du métier est en mesure de déterminer par l'intermédiaire des méthodes connues la quantité de glycine bétaïne présente dans la mélasse fermentée afin d'y ajouter l'acide selon le ratio molaire requis.

Cette étape d'acidification est ainsi mise en œuvre de manière à diminuer le pH de la mélasse fermentée de betterave à une valeur inférieure au pKₐ de la glycine bétaïne et des acides carboxyliques présents dans ladite mélasse.

La mélasse fermentée acidifiée présente ainsi un pH pouvant être compris de 1,1 à 1,7, et de préférence à un pH compris de 1,2 à 1,6.

Selon un mode de réalisation particulier, l'acide mis en œuvre est l'acide sulfurique ou l'acide méthane sulfonique.

La troisième étape du procédé selon l'invention consiste en une étape d'estérification de la mélasse fermentée acidifiée par mélange avec au moins un alcool.

Cette étape de mélange met en œuvre au moins un alcool, c'est-à-dire un seul alcool ou un mélange d'alcool.

L'alcool est mis en œuvre selon une quantité en excès par rapport à la glycine bétaïne. Ainsi, l'alcool est avantageusement ajouté selon un ratio molaire alcool/glycine bétaïne compris entre 1 et 2,5, de préférence compris entre 1,5 et 2,3.

Selon cette étape, le mélange est réalisé dans des conditions permettant l'obtention d'une réaction d'estérification entre les fonctions acides carboxyliques de la glycine bétaïne contenue dans la mélasse fermentée acidifiée et l'au moins un alcool mis en œuvre.

En d'autres termes, cette étape permet d'obtenir des esters de bétaïne à partir de la glycine bétaïne présente au sein de la mélasse fermentée et acidifiée de betterave.

L'estérification étant une réaction bien connue de l'homme du métier. Les conditions du mélange pour obtenir une telle réaction sont donc aisément adaptées par ce dernier.

Par exemple, le mélange de la mélasse fermentée acidifiée selon l'invention avec au moins un alcool peut être mis à reflux à une température comprise de 100°C à 120 °C, et pendant une durée comprise de 2h à 3h.

L'alcool mis en œuvre a une influence sur l'ester de bétaïne qui est obtenu. L'homme du métier est en mesure de choisir l'alcool ou le mélange d'alcool de manière à obtenir le ou les ester de bétaïne souhaités.

Selon un mode de réalisation particulier, l'alcool mis en œuvre est choisi dans le groupe comprenant l'éthanol, le glycérol, l'alcool laurylique(dodécan-1-ol), l'alcool isoamylique (3-méthylbutan-1-ol), l'alcool oléylique, l'alcool stéarylique, les alcools de fusel et leurs mélanges. De préférence, l'alcool est l'éthanol, l'alcool oléylique ou l'alcool laurylique.

Les alcools de fusel sont un mélange d'alcools supérieurs et inférieurs, alcools gras, terpènes et furfural. Ils se forment par la fermentation alcoolique comme sous-produits du métabolisme.

Selon un autre mode de réalisation particulier, l'alcool mis en œuvre est un alcool gras ayant une chaine C₃ à C₃₀, saturés ou insaturés comme par exemple l'octanol, le nonanol, l'undecanol, le dodecanol, ou encore le tridecanol.

Selon ce mode de réalisation particulier, la réaction d'estérification entre la mélasse fermentée et l'alcool gras génère deux phases distinctes. Une phase gel présentant un aspect pâteux et comprenant les esters de glycine bétaïne, et une phase liquide comprenant l'alcool gras en excès qui n'a pas réagi.

Le procédé selon l'invention permet ainsi d'estérifier la glycine bétaïne directement contenue dans la mélasse fermentée en utilisant cette dernière comme milieu réactionnel, sans étape préalable d'extraction. En fin du procédé, une mélasse fermentée et estérifiée est donc obtenue.

Le procédé selon l'invention permet donc d'obtenir une mélasse fermentée de betterave comprenant un ou plusieurs esters de glycine bétaïne et des esters d'autres acides carboxyliques.

Selon un mode de réalisation particulier, lorsque la réaction d'estérification est terminée, le procédé selon l'invention comprend une étape de centrifugation. Avantageusement, et lorsque la mélasse fermentée mise en œuvre selon la première étape du procédé n'est pas une mélasse déminéralisée, cette étape de centrifugation permet d'éliminer les précipités et les sels minéraux sulfatés.

Après la centrifugation, le surnageant est récupéré puis concentré de manière à obtenir un taux de matière sèche compris de 50% à 80%, et de préférence un taux de matière sèche compris de 60% à 70%.

Selon un mode de réalisation particulier, le procédé selon l'invention comprend une étape de récupération des esters de glycine bétaïne. Cette étape de récupération peut être réalisée après l'étape d'estérification ou après l'étape de centrifugation.

Selon ce mode de réalisation particulier, la récupération peut avantageusement être réalisée par une extraction.

Un autre objet de la présente invention concerne une mélasse fermentée de betterave, ladite mélasse fermentée étant estérifiée et comprenant un ou plusieurs esters de glycine bétaïne.

La mélasse fermentée est qualifiée d'estérifiée, car elle comprend un ou plusieurs esters de glycine bétaïne.

La mélasse fermentée et estérifiée de betterave comprenant un ou plusieurs esters de bétaïne selon l'invention est susceptible d'être obtenue par le procédé décrit précédemment.

Un autre objet de l'invention concerne l'utilisation de la mélasse fermentée et estérifiée de betterave pour améliorer les propriétés tensioactives et/ou émulsifiantes d'une composition.

En raison des ester cationiques qu'elle contient, la mélasse fermentée et estérifiée de betterave selon l'invention est utilisable dans tout type de composition mettant classiquement en œuvre des esters cationiques.

Par exemple, la mélasse fermentée et estérifiée de betterave selon l'invention peut être utilisée en tant qu'antistatique pour composition détergente automobile, qu'agent moussant pour détergent liquide, qu'agent anticorrosion pour pipeline, pour des émulsions bitumeuses, en tant qu'antifongique et mouillant pour adjuvant phytosanitaire, ou encore en tant qu'agent moussant et antistatique dans les shampoings.

La mélasse fermentée est utilisée en complément ou en remplacement total ou partiel des émulsifiants et/ou tensioactifs d'origine pétrochimique généralement utilisés dans les compositions.

La mélasse fermentée et estérifiée selon l'invention couvre également une large gamme d'applications dans le domaine de l'émulsification, comme dans l'industrie du pétrole, dans l'industrie des peintures, des pigments et des vernis ou encore dans l'industrie du bâtiment et des travaux publics.

L'invention sera mieux comprise à l'aide des exemples de réalisation et des figures ci-après, lesquels se veulent purement illustratifs et ne limitent en rien la portée de la protection.

### Figures

**Fig. 1**

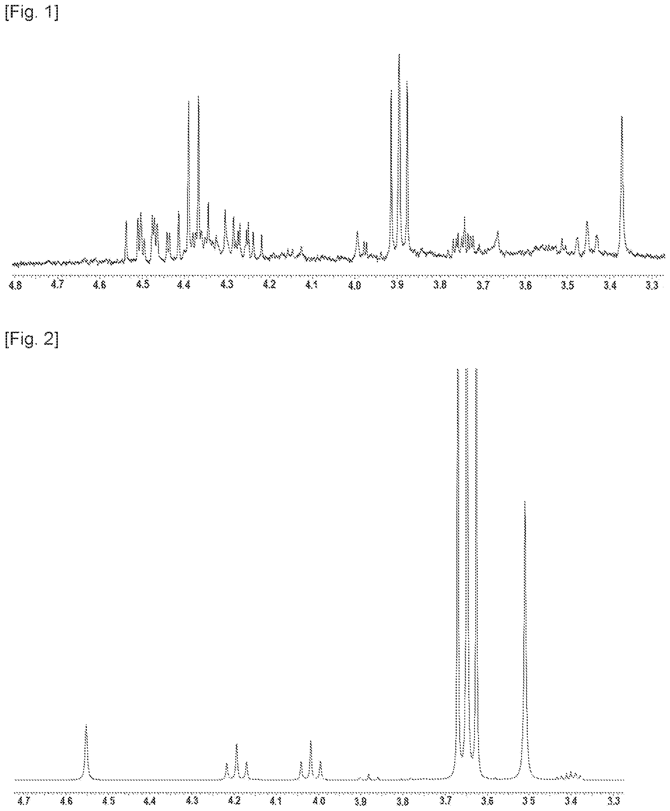

   [Fig. 1] Spectre RMN ¹H (CDCl₃) d'une solution de mélasse fermentée et supplémentée en glycine bétaïne.
**Fig. 2**
   [Fig. 2] Spectre RMN ¹H (CDCl₃) d'une fraction d'ether diéthylique contenant l'ester lauryl bétaïnate.
**Fig. 3**

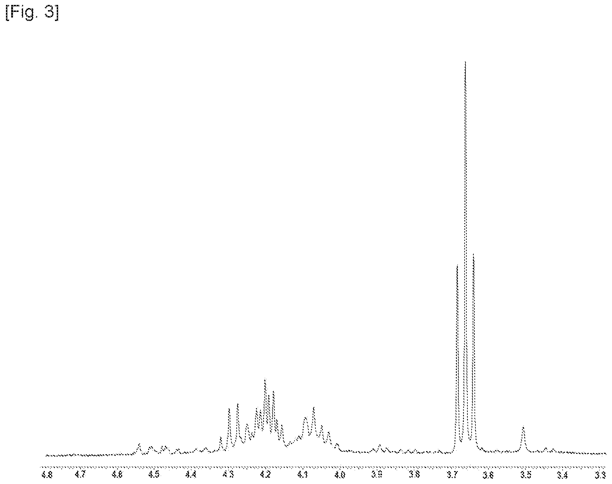

   [Fig. 3] Spectre RMN ¹H (CDCl₃) de mélasse fermentée et estérifiée avec l'alcool laurylique en présence d'H₂SO₄.
**Fig. 4**

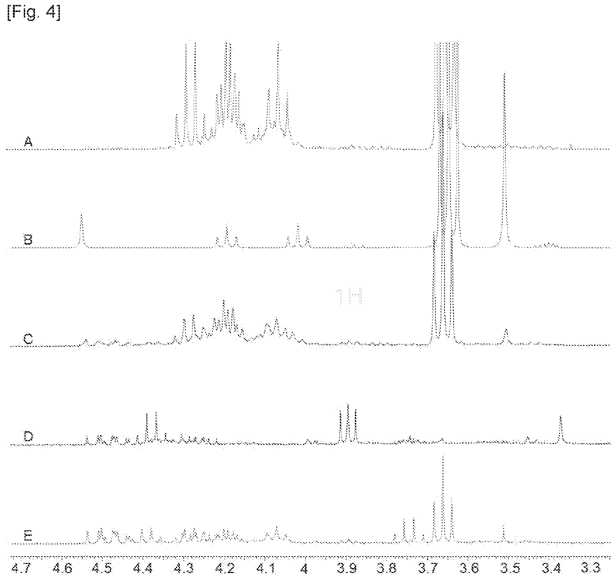

   [Fig. 4] Superposition de spectres RMN ¹H. **A** : Spectre RMN ¹H (CDCl₃) de la phase liquide de la mélasse fermentée et estérifiée avec un alcool oléylique ; **B** : Spectre RMN ¹H de d'une solution éther diéthylique contenant le lauryl bétaïnate ; **C** : Spectre RMN ¹H (CDCl₃) d'une mélasse fermentée et estérifiée avec un alcool laurylique en présence d'H₂SO₄ (contrôle); **D :** Spectre RMN ¹H (CDCl₃) d'une solution de mélasse fermentée et supplémentée en glycine bétaïne (contrôle) ; **E :** Spectre RMN ¹H (CDCl₃) de la phase gel de la mélasse fermentée et estérifiée avec un alcool oleylique.

### Exemples

### Exemple 1 : Préparation d'ester de glycine bétaïne à partir d'alcool laurylique (C12).

La mélasse fermentée utilisée pour cet exemple, est une mélasse déminéralisée présentant les caractéristiques suivantes :
- pH 3,
- 78% en poids de matière sèche par rapport à la matière sèche totale,
- environ 19% en poids de glycine bétaïne par rapport au poids total de la mélasse fermentée.

Une quantité de cette mélasse fermentée est acidifiée sous agitation constante dans un ballon de 250 mL avec 2,4 équivalents molaires d'acide sulfurique (concentré à 96%) par rapport à la glycine bétaïne. L'ensemble est homogénéisé.

L'alcool laurylique est ensuite ajouté dans le ballon selon un ratio molaire alcool/glycine bétaïne de 1,5 et l'ensemble est de nouveau homogénéisé.

Le ballon est placé sous évaporateur rotatif à chaud (90 °C) et sous pression réduite (100 mbar) avec une agitation de 100 à 150 rpm.

Après 3 heures, la réaction est arrêtée par trempage du ballon dans l'eau glacée.

En fin de réaction, la mélasse fermentée et estérifiée comprenant des esters de glycine bétaïne présente un aspect homogène.

Afin de confirmer la présence d'ester de glycine bétaïne dans la mélasse fermentée et estérifiée, une analyse RMN ¹H dans le chloroforme deutéré (CDCl₃) est réalisée et le résultat est comparé à des solutions contrôles.

Les solutions analysées en RMN ¹H sont reprises ci-dessous :
1 : Mélasse fermentée de betterave enrichie en glycine bétaïne (contrôle),
2 : Solution d'éther diéthylique contenant l'ester lauryl bétaïnate (contrôle),
3 : Mélasse fermentée et estérifiée avec l'alcool laurylique en présence d'H₂SO₄.

Les résultats des analyses RMN ¹H de chacune des solutions 1 à 3 sont respectivement présentés aux figures 1 à 3.

Le spectre contrôle de la figure 1 présente un pic à 3,37 ppm caractéristique de la glycine bétaïne.

Le spectre de la figure 2 sert également de contrôle et d'identifier le pic caractéristique de la glycine bétaïne estérifié par l'alcool laurylique (le lauryl bétaïnate) à 3,51 ppm, ainsi que trois pics entre 3,6 et 3,7 ppm caractéristiques de l'alcool laurylique.

Le spectre obtenu avec la mélasse fermentée et estérifiée présente un pic à environ 3,5 ppm et confirme la présence de l'ester lauryl bétaïnate. De plus, l'absence de pic caractéristique à 3,37 ppm permet de dire que la majorité de la glycine bétaïne a réagi pendant la réaction d'estérification pour former les esters de glycine bétaïne (Figure 3).

Ensembles, ces résultats confirment que le procédé selon l'invention permet d'obtenir des esters de glycine bétaïne à partir de mélasse fermentée, ladite mélasse fermentée étant utilisée directement en tant que milieu réactionnel.

### Exemple 2 : Préparation d'ester de glycine bétaïne à partir de l'alcool oléylique (C18).

La mélasse fermentée utilisée pour cet exemple est une mélasse déminéralisée présentant les caractéristiques suivantes :
- pH 3,
- 78% en poids de matière sèche par rapport à la matière sèche totale,
- environ 19% en poids de glycine bétaïne par rapport au poids total de la mélasse fermentée.

Une quantité de cette mélasse fermentée est acidifiée avec de l'acide sulfurique (concentré à 96%) sous agitation constante dans un ballon de 250 mL selon un ratio molaire acide/glycine bétaïne égal à 2, puis l'ensemble est homogénéisé.

Ensuite, l'alcool oléylique est ajouté dans le ballon selon un ratio molaire alcool/glycine bétaïne égal à 1,5, puis l'ensemble est de nouveau homogénéisé.

Le ballon est placé sous évaporateur rotatif à chaud (90 °C) et sous pression réduite (100 mbar) avec une agitation de 100 à 150 rpm.

Après 5 heures, la réaction est arrêtée par trempage du ballon dans l'eau glacée.

En fin de réaction, la mélasse fermentée et estérifiée comprenant des esters de glycine bétaïne se présente en deux phases, une phase gel et une phase liquide.

Afin de confirmer la présence d'ester de glycine bétaïne dans la mélasse fermentée et estérifiée, des analyses RMN ¹H sont réalisées sur les différentes phases obtenues et les résultats sont comparés à des solutions contrôles.

Les solutions analysées sont reprises ci-dessous :
**A :** Phase liquide de la mélasse fermentée et estérifiée avec un alcool oleylique,
**B :** Solution éther diéthylique contenant le lauryl bétaïnate,
**C :** Mélasse fermentée et estérifiée avec un alcool laurylique (C12) en présence d'H₂SO₄ (contrôle).
**D :** Solution de mélasse fermentée et supplémentée en glycine bétaïne (contrôle),
**E :** Phase gel de la mélasse fermentée et estérifiée.

Les résultats des analyses RMN ¹H de chacune des solutions A à E sont présentés en Figure 4.

Le spectre D permet d'identifier la position du pic caractéristique de la glycine bétaïne à 3,37 ppm.

Les esters de glycine bétaïne présentent sensiblement le même déplacement chimique quel que soit l'alcool utilisé pour l'estérification. Par conséquent, les spectres B et C sont utilisés comme contrôles avec le pic caractéristique de l'ester lauryl bétaïnate à 3,51 afin d'identifier la présence de l'ester oleyl bétaïnate.

L'absence du pic caractéristique de la glycine bétaïne sur les spectres A et E indique qu'elle a été en majorité consommée au sein de la mélasse fermentée lors de la réaction d'estérification pour former les esters de glycine bétaïne. Les esters oleyl bétaïnate se retrouvent uniquement dans la phase gel de la mélasse fermentée et estérifiée comme en atteste la présence du pic à 3,51 ppm sur le spectre E, ledit pic étant absent sur le spectre A de la phase liquide.

De nouveau, les résultats confirment qu'il est possible d'obtenir des esters de glycine bétaïne à partir de mélasse fermentée, ladite mélasse fermentée étant utilisée directement en tant que milieu réactionnel.

Allant ainsi à l'encontre de ce qui été jusqu'à présent admis, la Demanderesse prouve qu'il est possible de réaliser une réaction d'estérification à partir de la mélasse fermentée obtenant ainsi des esters de glycine bétaïne après ajout d'alcool.

### Exemple 3 : Préparation d'ester de glycine bétaïne à partir de mélasse fermentée et d'éthanol.

Un volume de mélasse fermentée de betterave est acidifié avec de l'acide sulfurique jusqu'à pH 1,9.

La mélasse fermentée acidifiée est ensuite mélangée soit avec 1,1 équivalent molaire d'éthanol par rapport à la glycine bétaïne (mélange 1), soit avec 5% en poids de glycérol (mélange 2).

Les deux mélanges sont mis à reflux à 110 °C pendant 2h30.

Ensuite, une étape de centrifugation est mise en œuvre à 20 °C pendant 10 min à 8000 tr/min afin d'éliminer les précipités et les sels minéraux sulfatés. Le surnageant est récupéré puis concentré par évaporation jusqu'à obtenir une matière sèche d'environ 65% et la présence d'esters de glycine bétaïne a été confirmée par mesures RMN 1H.

## Revendications

1. Procédé de préparation de mélasse fermentée comprenant au moins un ester de glycine bétaïne, ledit procédé comprenant les étapes suivantes de :
1) fourniture d'une mélasse fermentée de betterave,
2) ajout dans ladite mélasse fermentée de betterave d'au moins un acide selon un ratio molaire acide/glycine bétaïne compris entre 1 et 2,2,
3) estérification de la mélasse fermentée acidifiée obtenue à l'étape précédente par mélange avec au moins un alcool.

2. Procédé selon la revendication 1, **caractérisé en ce que** la mélasse fermentée de betterave est un mélange de mélasse fermentée de betterave et de mélasse fermentée de canne.

3. Procédé selon la revendication 1 à 2, **caractérisé en ce que** l'alcool est mélangé avec la mélasse fermentée acidifiée selon un ratio molaire alcool/glycine bétaïne compris entre 1 et 2,5.

4. Procédé selon l'une des revendications 1 à 3, **caractérisé en ce que** l'alcool est choisi parmi l'éthanol, le glycérol, l'alcool laurylique(dodécan-1-ol), l'alcool isoamylique (3-méthylbutan-1-ol), l'alcool oléylique, l'alcool stéarylique, les alcools de fusel et leurs mélanges, de préférence parmi l'éthanol, l'aclool oléylique et l'alcool laurylique.

5. Procédé selon l'une des revendications 1 à 4, **caractérisé en ce qu'**il comprend après l'étape 3) d'estérification, une étape de centrifugation et de concentration du surnageant à un taux de matière sèche compris de 50% à 80%.

6. Procédé selon l'une des revendications 1 à 5, **caractérisé en ce que** la mélasse fermentée de betterave fournie selon la première étape est une mélasse fermentée et déminéralisée.

7. Mélasse fermentée et estérifiée de betterave comprenant un ou plusieurs esters de glycine bétaïne.

8. Utilisation de la mélasse fermentée telle que revendiquée à la revendication 7 pour améliorer les propriétés tensioactives et/ou émulsifiantes d'une composition.

## Patentansprüche

1. Verfahren zur Herstellung von fermentierter Melasse, umfassend wenigstens einen Glycinbetainester, wobei das Verfahren die folgenden Schritte umfasst:
1) Bereitstellen einer fermentierten Rübenmelasse,
2) Zugabe wenigstens einer Säure zu der fermentierten Rübenmelasse mit einem Molverhältnis Säure/Glycinbetain zwischen 1 und 2,2,
3) Veresterung der in dem vorhergehenden Schritt erhaltenen angesäuerten fermentierten Melasse durch Vermischen mit wenigstens einem Alkohol.

2. Verfahren nach Anspruch 1, **dadurch gekennzeichnet, dass** die fermentierte Rübenmelasse ein Gemisch aus fermentierter Rübenmelasse und fermentierter - Zuckerrohrmelasse ist.

3. Verfahren nach Anspruch 1 bis 2, **dadurch gekennzeichnet, dass** der Alkohol mit der angesäuerten fermentierten Melasse in einem Molverhältnis Alkohol/Glycinbetain zwischen 1 und 2,5 gemischt wird.

4. Verfahren nach einem der Ansprüche 1 bis 3, **dadurch gekennzeichnet, dass** der Alkohol gewählt ist aus Ethanol, Glycerin, Laurylalkohol (Dodecan-1-ol), Isoamylalkohol (3-Methylbutan-1-ol), Oleylalkohol, Stearylalkohol, Fuselalkohole und deren Gemische, vorzugsweise aus Ethanol, Oleylalkohol und Laurylalkohol.

5. Verfahren nach einem der Ansprüche 1 bis 4, **dadurch gekennzeichnet, dass** es nach dem Schritt 3) der Veresterung einen Schritt des Zentrifugierens und Konzentrierens des Überstands auf einen Trockensubstanzgehalt von 50 % bis 80 % umfasst.

6. Verfahren nach einem der Ansprüche 1 bis 5, **dadurch gekennzeichnet, dass** die in Schritt 1 bereitgestellte fermentierte Rübenmelasse eine fermentierte und entmineralisierte Melasse ist.

7. Fermentierte und veresterte Rübenmelasse, die einen oder mehrere Glycinbetainester enthält.

8. Verwendung der fermentierten Melasse nach Anspruch 7 zur Verbesserung der oberflächenaktiven und/oder emulgierenden Eigenschaften einer Zusammensetzung.

## Claims

1. A process for the preparation of fermented molasses comprising at least one glycine betaine ester, said process comprising the following stages of:
1) provision of a fermented beet molasses,
2) addition, to said fermented beet molasses, of at least one acid according to an acid/glycine betaine molar ratio of between 1 and 2.2,
3) esterification of the acidified fermented molasses obtained in the preceding stage by mixing with at least one alcohol.

2. The process as claimed in claim 1, **characterised in that** the fermented beet molasses is a mixture of fermented beet molasses and fermented cane molasses.

3. The process as claimed in claim 1 or claim 2, **characterised in that** the alcohol is mixed with the acidified fermented molasses according to an alcohol/glycine betaine molar ratio of between 1 and 2.5.

4. The process as claimed in one of claims 1 to 3, **characterised in that** the alcohol is chosen from ethanol, glycerol, lauryl alcohol (dodecan-1-ol), isoamyl alcohol (3-methylbutan-1-ol), oleyl alcohol, stearyl alcohol, fusel alcohols and their mixtures, preferably from ethanol, oleyl alcohol and lauryl alcohol.

5. The process as claimed in one of claims 1 to 4, **characterised in that** it comprises, after the esterification stage 3), a stage of centrifugation and concentration of the supernatant to a dry matter content of from 50% to 80%.

6. The process as claimed in one of claims 1 to 5, **characterised in that** the fermented beet molasses supplied according to the first stage is a fermented and demineralized molasses.

7. A fermented and esterified beet molasses comprising one or more glycine betaine esters.

8. Use of the fermented molasses as claimed in claim 7 for improving the surfactant and/or emulsifying properties of a composition.
